Europäisches Patentamt

(19) European Patent Office

Office européen des brevets

(11) Publication number: **0 158 107**
A2

(12) **EUROPEAN PATENT APPLICATION**

(21) Application number: 85102420.8

(22) Date of filing: 05.03.85

(51) Int. Cl.⁴: **A 61 M 25/00**
**A 61 M 27/00**

(30) Priority: 05.03.84 YU 400/84

(43) Date of publication of application:
16.10.85 Bulletin 85/42

(84) Designated Contracting States:
AT BE CH DE FR GB IT LI LU NL SE

(71) Applicant: Stakic, Bogdan Dr.
Carli Caplina 40
11 000 Beograd(YU)

(72) Inventor: Stakic, Bogdan Dr.
Carli Caplina 40
11 000 Beograd(YU)

(74) Representative: Haft, Berngruber, Czybulka
Hans-Sachs-Strasse 5
D-8000 München 5(DE)

(54) Hydraulic, soft, permanent catheter.

(57) A hydraulic soft permanent catether belongs to medical technical items, or, more precisely is utilized for fluid discharge from human body. Permanent catether, now in use, is rigid one and because of that may cause damage while carried and changed, favouring infection.

A hydraulic soft permanent catether made of medical menmade has great advantiges over a standard rubber catether:
urinary outflow bettered for 30%, diminishes uretral injuries, and produces no injuries of back wall of the bladder, enhences draneige of catether uretral space, diminishes residual urine as well as necessity for frequent change of the catether.

Technological procedure is much more simplified and cheaper.

FIG 1.

EP 0 158 107 A2

Stakic, Bogdan, MD
40 Carli Caplina
11000 Beograd
YUGOSLAVIA

0158107

## I. HYDRAULIC, SOFT, PERMANENT CATHETER

### A. Technical Area of Invention

1. Hydraulic, soft, permanent catheter belongs to the area of medical technology dealing with extraction of fluids from the body.

### B. Technical Problem

1. Through the solution of this technical problem, it has become possible to use a catheter which is by far softer than presently used ones, as well as avoiding requirement of frequent replacements of the same, due to plugging. As a consequence of the aforementioned, use of hydraulic, soft, permanent catheter diminishes injuries and infections and thus causes less difficulties.

### C. Present State of Technique

1. Permanent catheter presintly in use is made out of rubber (hard red rubber, Latex-softer rubber or silicon rubber-silastic), hard Neplex plastic etc. All these materials are sufficiently rigid to permit insertion of catheter into bladder without the use of mandrin or other supportive aids.

2. Permanent catheter is about 40 cm long tube, 12 to 24 charriere-s thick. Charriere is an international unit for catheter thickness measurement. One millimeter of thickness corresponds to 3 Charriere-s. The external segment of permanent catheter ends with a conic enlargement for the purpose of connecting it to the tube leading to a urine bag or or connecting it to the tip of Janett syringe. Janett syringe has a volume of over 50 cc and a conic tip (male) so that connection to the catheter is easy and it is used for catheter rinsing. Also, at the external segment there is a stem with a valve for inflating control balloon. At the internal end of the catheter there is a balloon made of thin rubber. Five ten cubic centimeters in volume which is to be inflated with fluid via a separate lead which at the external side ends up with already mentioned valve. From balloon outward, cateter continues toward the tip where it ends either with a blunted tip and an opening on the side (Foley-type) or with two opening opposite each other

0158107

(Harris-type) or with a bent pointed tip (Timann-type).

D. Shortcomings of Presently Produced Permanent Catheter

1. Rubber surface is coarse and is being "wetted" by liquid, which leads to salt deposition from urine. Consequently such permanent catheters must be changed every 2 to 3 weeks. Silicone-rubber catheters last somewhat longer.

2. Because of the unevenness of rubber wall, urine flow through catheter is lower and when catheters are thick, with high Charriers, the spacing between mucosa of urethra and catheter is small. Consequently, the drainage of detritus and mucus from urethra's mucosa becomes more difficult and speeds up development of an infection.

3. Due to hardness, permanent catheter harms urethra's wall while the tip located inside bladder is harming rear wall of the bladder.

4. Opening of the permanent catheter is located about 1 cm above Balloon so that only partial outflow of urine is possible and part of urine remains permanently in the bladder, beside permanent catheter.

5. Due to catheter hardness and following an infection, patients hardly can bear the pain and when complications take place (stenosis, uretritis, abscess, etc) lead them to desperation.

E. Description of Technical Solution of the Problem with Application Example and Brief Design Description.

1. Hydraulic, soft, permanent catheter is constructed out of soft medical plastic. Plastic is so soft that catheter by its own stiffness can not be inserted into urinary bladder. Since plastic is hydrophobic and the surface glossy, it does not get "wetted" by passing urine, which improves the flow up to 30%, in comparison with rubber catheter of the same size. For this reason, it is possible to use a catheter with smaller Charrieres, without altering urine flow velocity through the catheter. It follows, that the spacing between catheter wall and urethra wall would be increased. As a rule, catheter thickness would remain the same as of the presently made permanent catheters. Though the width of 18 Charrieres is considered optimal. Standard catheter length is approximately 40 cm. The loose end is conically enlarged (female) in the same fashion as is presently done with standard catheters, for the purpose of connecting it to a urine bag or Janett syringe. Frontal, internal part can be constructed in several different ways. The diagrams illustrate this point:

   Type I, Page 1, Sketch 1 Devite is made up of the body of the catheter, approximately 40 cm long (1), at the rear end it is conically enlarged (7) circumferential enlargement on front end (2) and openings (3,4) on the flat head surface. A

0158107

cylinder (5) with centrally located release valve (6) is clipped and inserted into tube (1). Elastic lamella (8) is located at the rear end of the cylinder (5). Cylinder is tied with a plastic thread (9) so that it can be pulled out after its use. Diagram 2 depicts front side of the circumferential enlargement. Cross-section A-A through the catheter body is shown on diagram 3. Page 2, diagram 4 merely magnifies circumferential enlargement (2), with openings (3) and (4), sliding cylinder (5) with central release valve (6).

Type II, Page 3, Diagram 5.

According to this type, mid and rear parts of the catheter are the same, while front part is different. Circumferential enlargement (9) is in front part, with the end (11) pulled in toward the rear. From the head side of the hollow tubing (13) an elastic ball (12) is slipped inside. This ball is tied with a plastic thread so that it can be readily pulled out. Bottom view of the circumferential enlargement is shown on diagram 6. Cross-section A-A of the catheter is shown on diagram 7. Page 4, diagram 8 reflects magnified circumferential enlargement (9) with the end (11) pulled in toward the rear.

Catheter is used, by initially injecting sterilized liquid with a syringe at the external and of the catheter. When sufficient liquid pressure is built up, catheter stiffens since plastic does not expend like rubber. Further increments in liquid pressure from the syringe activate hydraulic cylinder (Type I) or move the spherule (Type II), in such a manner that circumferential enlargement gets elongated at the front end of the catheter, thus directionally acquiring the shape of a spindle, which is a satisfactory form for insertion into urinary bladder. In front of the syringe then one clamps a hemostat, syringe is separated from catheter and so stiffened is introduced into urinary bladder. As the hydraulic, soft, permanent catheter is inserted in urinary bladder, hemostat is released and cylinder or spherule fall out or or are pulled out by thread. When pressure drops, circumferential enlargement is restored into its initial state. Which does not permit slipping out of catheter from the urinary bladder. When cather is to be taken out, some procedure is used but in reverse order.

F. Reported Advantages of the Aforementioned Catheter

1. Since plastic wall is glossy and hydraphobic, there are no deposits of urinary salts, so that catheter replacement is not as frequent.
2. Due to wall smoothness, urine flow is easier and that permits the use of a thinner catheter.
3. Spacing between epithelium of urethra and catheter wall diminishes the possibility of infection development.

4. Due to the catheter softness the possibility of injury to urethra is reduced and since there is no tip to the catheter rear wall of urinary bladder can not be hurt.
5. Since catheter opening is at the lowest position of the urinary bladder, residual urine is minimal.
6. Simplified manufacturing and production, in comparison with standard permanent catheter.

Some difficulties may arise with the aforementioned catheter due to relatively complicated implantation procedure of the catheter into urinary bladder. In the case of bladder hemorrhaging, extraction of the coagulum may become impossible of the catheter wall is excessively soft.

Stakić dr Bogdan
Čarli Čaplina 40
11000 BEOGRAD

PATENT DEMAND

1.A hydraulic soft permanent catether,according to variant I consists of menmade tube of corresponding lenght,caracterized by radial enlargening /2/, holes /3,4/, on front side,flexibile tube /1/, having conical widening on opposite side /7/, with sliding valve /5/, with central prolonged part /6/, with elastic lamella fixed to back wall of the valve,and pull out thread /9/.

2.A hydraulic soft permanent catether,according to variant II, for permanent discharge of urine from the bladder,composed of men made tube of corresponding lenght,caracterized by radial enlargement /9/, backwardly tracted end /11/,on front side derived tubes /13/, is slidingly incorporated ball /12/.

Stakić dr. Bogdan
Čarli Čaplina 40
11000 BEOGRAD

7

Section A-A

Fig. 3

View A

9

1

8

5

6

2

3

A

4

3

Fig. 2

Fig. 1

HYDRAULIC, SOFT
PERMANENT CATHETER

0158107
pages 4

Fig. 4

Dr Stakić
Bogdan

*HYDRAULIC, SOFT
PERMANENT CATHETER*

0158107   3/
pages   /4

7

Section A-A

Fig. 7

View A

14

13

A↑   ↑A

12

11

9

10

Fig. 5

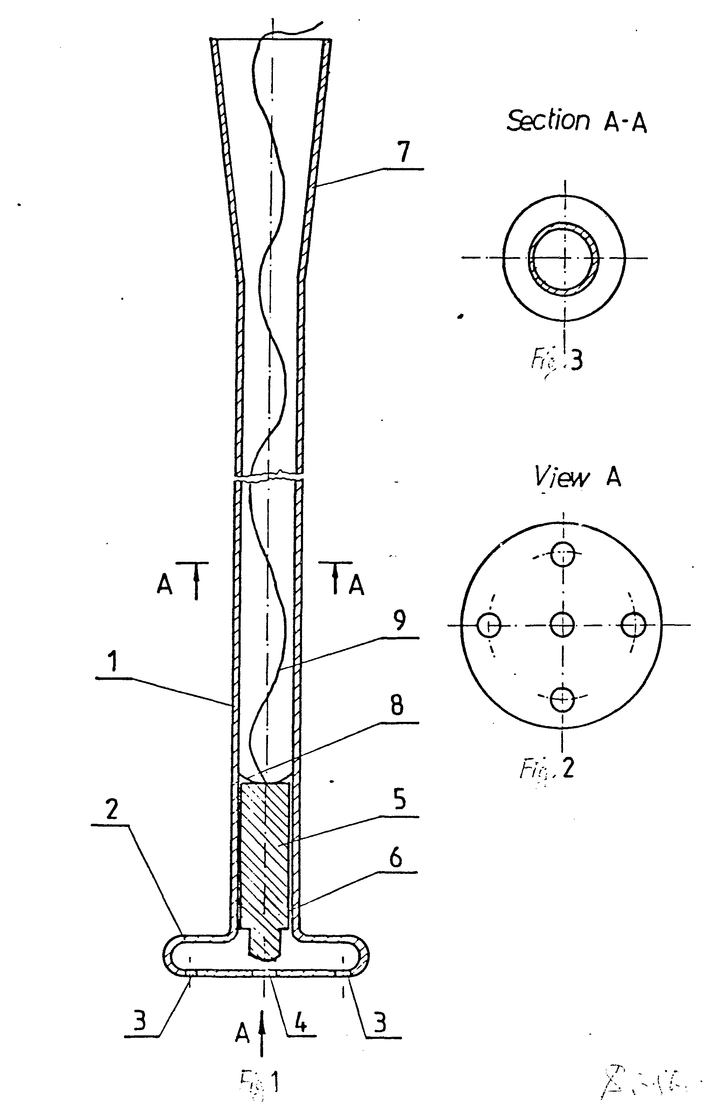

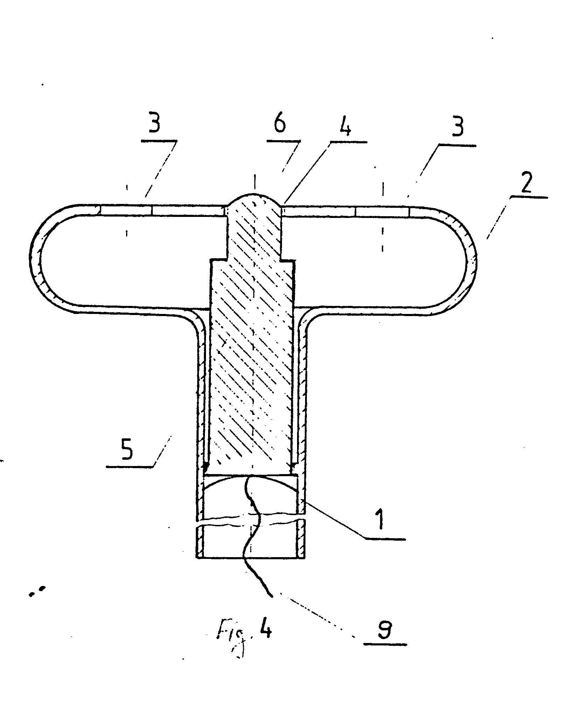

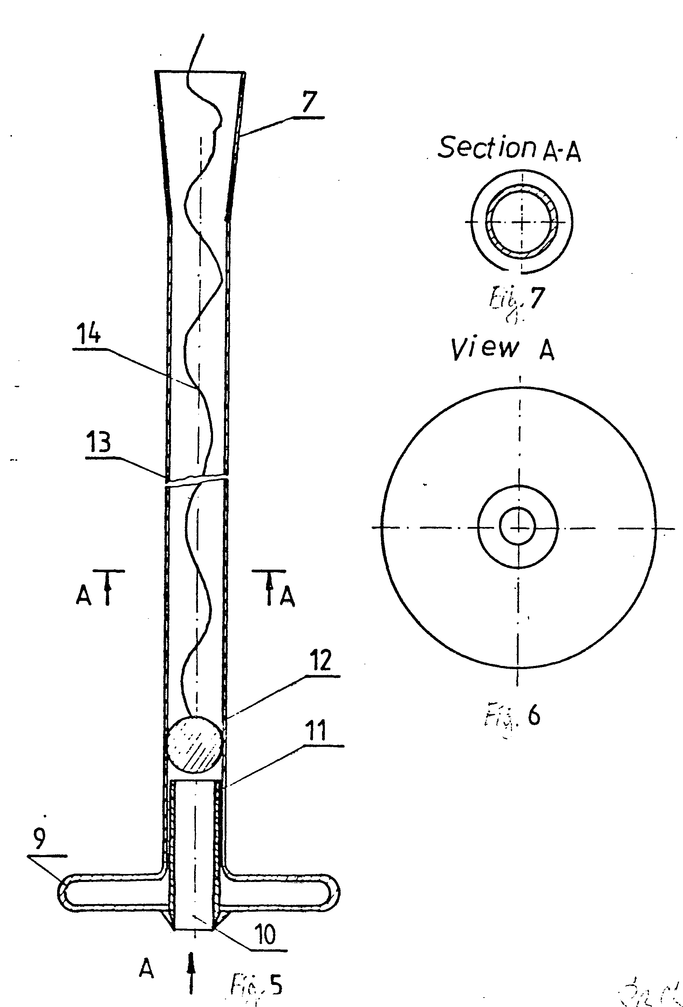

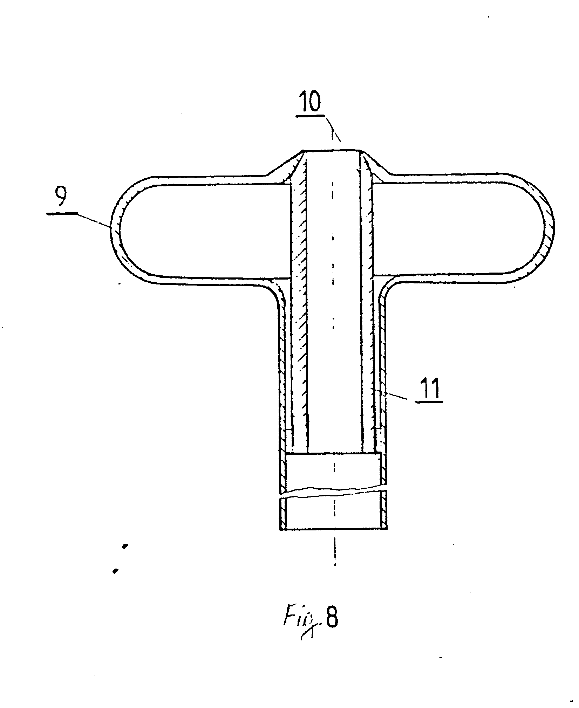

A↑

Fig. 6

r Sickić
Bogdan

HYDRAULIC, SOFT
PERMANENT CATHETER

0.158107

pages /4

10

9

11

Fig. 8